(19) Europäisches Patentamt
European Patent Office
Office européen des brevets

(11) **EP 2 929 336 B1**

(12) **EUROPEAN PATENT SPECIFICATION**

(45) Date of publication and mention of the grant of the patent:
**18.01.2017 Bulletin 2017/03**

(51) Int Cl.:
**G01N 27/327** *(2006.01)*    **G01N 33/50** *(2006.01)*
**G01N 33/66** *(2006.01)*

(21) Application number: **13799542.9**

(22) Date of filing: **03.12.2013**

(86) International application number:
**PCT/EP2013/075436**

(87) International publication number:
**WO 2014/086803 (12.06.2014 Gazette 2014/24)**

(54) **METHOD FOR HEMATOCRIT CORRECTION AND GLUCOSE METER ADAPTED THEREFOR**

VERFAHREN ZUR HÄMATOKRIT-BERICHTIGUNG UND DARAUF ANGEPASSTER GLUCOSEMESSER

PROCÉDÉ DE CORRECTION D'HÉMATOCRITE ET COMPTEUR DE GLUCOSE CONÇU À CET EFFET

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.12.2012 EP 12195570**

(43) Date of publication of application:
**14.10.2015 Bulletin 2015/42**

(73) Proprietors:
• **Roche Diabetes Care GmbH**
**68305 Mannheim (DE)**
Designated Contracting States:
**DE**
• **F. Hoffmann-La Roche AG**
**4070 Basel (CH)**
Designated Contracting States:
**AL AT BE BG CH CY CZ DK EE ES FI FR GB GR HR HU IE IS IT LI LT LU LV MC MK MT NL NO PL PT RO RS SE SI SK SM TR**

(72) Inventors:
• **SCHULAT, Jochen**
**68305 Mannheim (DE)**

• **TRICK, Sebastian**
**68239 Mannheim (DE)**

(74) Representative: **Pfiz, Thomas et al**
**Wolf Pfiz & Gauss**
**Patentanwälte**
**Hauptmannsreute 93**
**70193 Stuttgart (DE)**

(56) References cited:
**US-A1- 2004 225 205     US-A1- 2007 231 209**
**US-A1- 2010 243 476     US-B1- 6 602 469**

• **David A Lacher ET AL: "Biological Variation of Hematology Tests Based on the 1999-2002 National Health and Nutrition Examination Survey", National Health Statistics Reports, 31 July 2012 (2012-07-31), pages 1-12, XP055059289, Retrieved from the Internet: URL:http://198.246.98.21/nchs/data/nhsr/nhsr054.pdf [retrieved on 2013-04-11]**

**Description**

The invention concerns a method for hematocrit correction and a glucose meter according to claims 1 and 15, respectively. The invention further concerns a system comprising a portable glucose meter and a reference instrument.

The hematocrit (HCT) may be defined as the volume percentage (%) of red blood cells in whole blood. The HCT is normally about 45% for men and 40% for women and may range from about 20% to about 70% in extreme cases. It is known that the hematocrit can impact the glucose level of a blood sample being tested. In order to account for such a hematocrit interference, it has been proposed to additionally measure the actual hematocrit value of a given sample, e.g. by multiple wavelength, conductivity or other tests in addition to the glucose test. However, such measurements imply unwanted complexity in self-testing devices and are prone to measurement uncertainty. As an alternative, efforts have been made to reduce the hematocrit influence by the design of the test chemistry or disposable, e.g. by retaining red blood cells through separating layers. However, such a measure can eliminate the hematocrit influence only to a residual dependency.

US-A1-2012103806 (Shinno Teppei et al.), May 3, 2012, describes methods and instruments for measuring hematocrit. No hint directed to non-fluctuating hematocrit values can be derived from this document. US-A1-2004/225205 (Ilya Fine et al.), November 11, 2004, describes a method to measure hematocrit based on repeated measurements.

On this basis the object of the invention is to further improve the known methods and devices for hematocrit correction in glucose measurements and to provide improved measurement certainty specifically in a self-testing environment without undue effort.

The combination of features stated in the independent claims is proposed to achieve this object. Advantageous embodiments and further developments of the invention are derived from the dependent claims.

The invention is based on the finding that the mean/average hematocrit of a given person is (under normal life conditions) fluctuating only in a limited range. Accordingly it is proposed according to the invention that a method for hematocrit correction in a glucose meter comprises the steps of

- determining by means of a reference instrument preferably formed as a laboratory analyzer a hematocrit reference value of a reference blood sample taken from a specific user,
- applying a fresh blood sample of said user on a disposable analytical test element,
- measuring the glucose value of the fresh blood sample by single use of said test element in the glucose meter,
- determining a hematocrit correction value using at least the hematocrit reference value,
- adjusting the measured glucose value using the hematocrit correction value to receive an unbiased adjusted glucose value.

Such a procedure requires only once the determination of the hematocrit reference value, which can be exactly measured by use of a clinical or laboratory analyzer, whereas the routinely glucose measurements on the spot can be repeatedly conducted and corrected on the basis of one and the same hematocrit reference value without increased measurement effort. This is also due to the finding that the hematocrit dependency of typical self-monitoring blood glucose monitoring systems comprising a given test architecture and device is relatively constant. The adjustment of the measured glucose value can be easily implemented on processors which are already included in handheld devices or home meters for other data handling purposes. Thus, the system performance can be improved significantly, whereat the meter is then assigned to a specific user, i.e. as a personalized device. In this way, the hematocrit correction is easily feasible in a glucose monitoring system without the need for the patient to bring blood samples to a laboratory for determining the glucose bias in each and every case. Advantageous for a convenient handling, the hematocrit reference value may be transferred via a wireless or wire-bound interface into a memory of the glucose meter.

For safety considerations, it is further advantageous when the hematocrit reference value is transmitted to the glucose meter using an external software on a device outside the glucose meter which is inaccessible to the user.

A further improvement for convenience may be achieved when the hematocrit reference value is stored in an external database outside the glucose meter in connection with a user identifier for the user.

To facilitate data exchange for a personalized device, the glucose meter may comprise machine readable means, specifically an RFID chip, for automatic user identification.

Another safety improvement provides that the user identity is checked by a query provided by the glucose meter, whereupon an input of a confirmation by the user is requested.

In order to account for eventual deviations of the hematocrit reference value, the user may be queried about a change in living conditions influencing hematocrit.

For a reliability check it is also favorable when the timeliness of the hematocrit reference value is verified within a given time interval.

For further awareness of the patient or user, it is advantageous when the user is informed that personalized data are used for correction of the measured glucose value.

In order to avoid unwanted loss of a test medium, an advantageous embodiment provides that the adjusted glucose value is displayed to the user upon fulfillment of given conditions including availability of the hematocrit reference value and optionally timeliness of this value, whereas otherwise in order to provide a fall back result the measured glucose value is displayed.

It is also advantageous for improved elimination of the hematocrit effect when the hematocrit correction value is determined in dependence of the hematocrit reference value and the measured glucose value.

Advantageously, determining of the hematocrit correction value involves using one or more correction functions or a lookup table determined empirically in connection with the architecture of the test element eventually in combination with the glucose meter.

The hematocrit correction is particularly effective when the glucose value of the fresh blood sample is measured by photometric or electrochemical detection on the analytical test element.

Advantageously, the glucose meter is construed as a portable handheld device usable by a proband or user for self-testing in a non-laboratory environment.

With regard to a glucose meter adapted for hematocrit correction, in order to solve the aforementioned object, the following combination of features is proposed according to the invention:

- means configured to receive at least one disposable test element on which a blood sample can be applied or is applied,
- a detector adapted for measuring a blood glucose value using the test element loaded with a fresh blood sample of a specific user,
- an interface configured to input a hematocrit reference value of a reference blood sample of said user,
- a processor adapted to determine a hematocrit correction value using the hematocrit reference value and the measured glucose value and to adjust the measured glucose value using the hematocrit correction value.

For a trusted execution of the hematocrit correction it is advantageous to provide means operable to allow hematocrit correction of the blood glucose measurement depending on the provision of a (valid) hematocrit reference value. It may also be conceivable that in case of a missing hematocrit reference value an uncorrected measurement result is provided together with a corresponding indication to the user.

A further aspect of the invention comprises a system adapted for hematocrit correction, comprising the glucose meter according to the invention and a reference instrument preferably formed as a laboratory analyzer to determine a hematocrit reference value of a reference blood sample taken from a specific user of the glucose meter.

The invention is further elucidated in the following on the basis of embodiment examples shown schematically in the drawings, where

Fig. 1     is a perspective and partially schematic view of a glucose meter in connection with an external reference system for hematocrit correction;

Fig. 2     is a plot of hematocrit-induced glucose bias $\Delta$ versus the glucose concentration C for a given hematocrit value.

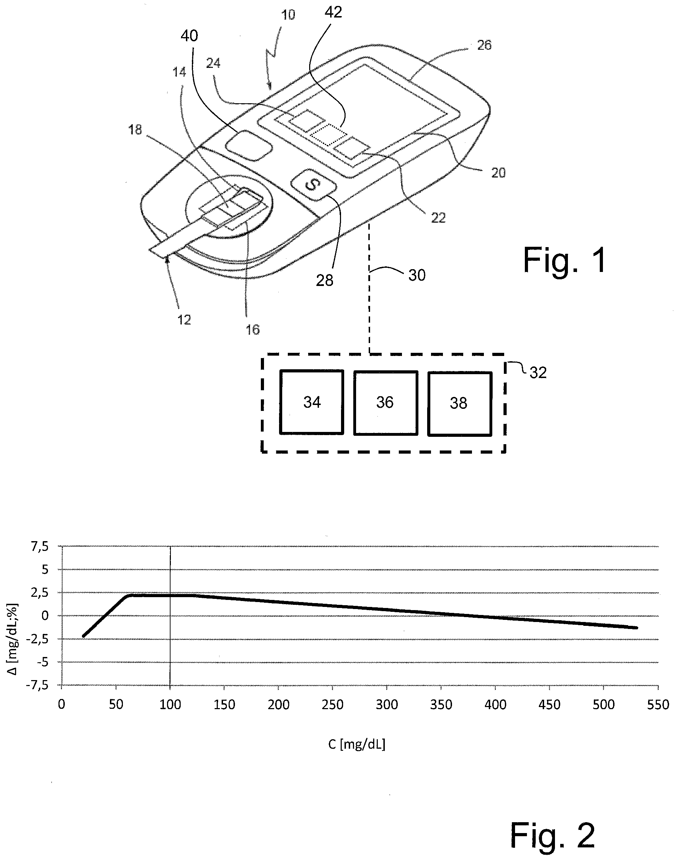

FIG. 1 illustrates an exemplary handheld glucose meter 10 for insertion of a disposable test strip 12 usable by a proband or user for self-testing in an everyday environment. The meter 10 comprises a holder 14 to position the test strip 12 in the optical path of a reflection-photometric detector 16 to read the reflectance of a test pad 18 of the strip 12. A small volume of a fresh sample of whole blood taken by the user on the spot can be applied to the test pad 18, wherein a reagent reacts with a glucose leading to a change in reflectance which is detectable from the bottom of the test pad 18 with the photometer 16. Such measurements are known to the skilled person per se and need not to be elucidated in more detail. It is further known that the hematocrit content of a blood sample can impact the glucose level to be tested e.g. by diffusion effects in the test pad 18.

In order to process and correct the measurement signals, a device electronics 20 comprises a processor 22, a memory 24, a display 26 and keys 28 for interaction with the user and an interface 30 for eventual connection to an external reference system 32. The processor 22 is adapted for hematocrit correction using the measured glucose value and a hematocrit reference value initially provided through the reference system 32 and stored in the memory 24.

The hematocrit reference value can be determined by means of an external reference instrument 34 formed as a laboratory analyzer. For this purpose, a specific user may provide a reference blood sample to be analyzed with the reference instrument 34 in a clinical or laboratory setting. Then, the determined hematocrit reference value can be transmitted into the memory 24 of the glucose meter 10 via the (wireless) interface 30 using an external software 36 running on a device outside the meter 10. In order to guarantee a safe handling, the software 36 should be inaccessible to the user and only operable by authorized personnel, e.g. by a health professional. For example, a physician may connect the glucose meter 10 of a patient to a computer in his medical practice running the software 36 such that configuration data of the meter 10 can be read out and the hematocrit reference value can be set only by the physician, to thereby ensure that the values are controlled and interpreted with the necessary medical knowledge and are not manipulated by a layperson.

It may also be conceivable that the hematocrit reference value is stored in a database 38 of the reference system 32 in connection with an identifier for the user who has provided the reference sample. An automatic data transfer to the glucose meter 10 assigned to said user could then be accomplished by an identification process enabled by machine readable means, specifically an RFID chip 40 mounted on the meter 10 and containing the user identifier.

It should be emphasized that such an initial procedure is only necessary once in a while, as the hematocrit value of a given patient is usually relatively constant over time. Given the living situation does not change, the hematocrit value of an individual typically fluctuates by less than 2%, which is small compared to the possible range of hematocrit values for different persons (typically 20 to 55%, eventually up to 70%).

By storing the hematocrit reference, the meter 10 is personalized for the specific user and can be employed for glucose measurements in a daily routine. In order to carry out such a measurement, the user takes a fresh blood sample and applies it on the test strip 12 before or after insertion into the meter 10, in which a glucose value can be measured automatically by means of the detector 16. At the beginning of the measurement routine, the user identity is checked e.g. by a query displayed to the user on the display 26 and requesting input of a confirmation by means of keys 28. The user can be informed by an indication on the display 26 that personalized data are used for correction of the glucose measurement. The user may further be asked about a change in living conditions which may influence the hematocrit, for example training in higher altitudes.

The processing routine may also include a verification of the timeliness of the hematocrit reference value, which should be updated regularly, e.g. once in a year.

The meter 10 may comprise an activation stage 42 e.g. in the form of a software routine or input field to allow a glucose measurement only if a valid hematocrit reference value is available. The validity and specifically the attribution to a specific user may be proved by a security query to be confirmed by the user. Alternatively, in case of a missing hematocrit reference value, the processing routine could provide the measured glucose value to the user together with information that no correction has been made.

If a valid hematocrit reference value is stored in the memory 24, the hematocrit correction value is determined in dependence of the hematocrit reference value and the measured glucose value. Then, the measured glucose value is adjusted using the hematocrit correction value to receive an adjusted glucose value unbiased by hematocrit.

The measured glucose concentration can be corrected in consideration of the hematocrit reference value by using one or more correction functions. For example, a correction function in the form of a correction equation may be used, in which one or more correction factors and/or one or more correction offsets are used. It has been found that the correction of the measured glucose concentration C(meas) can be effected for example according to the following equation:

$$C(corr) = C(meas) + m*HCT^i + n \qquad (1)$$

In this equation, HCT is the hematocrit reference value, C(meas) is the measured glucose concentration, C(corr) is the corrected glucose concentration, and the factor m and the exponent i are experimentally or empirically determined correction parameters, which may, for example, depend on the temperature and the concentration of glucose itself.

Fig. 2 illustrates the deviation $\Delta$ of the measured glucose concentrations from the actual glucose concentrations C(ref) determined by a means of a reliable reference method. The uncorrected glucose concentration could be measured using the handheld glucose meter 10, and the actual glucose concentrations could be determined using a laboratory device, or in other ways. For a sample with a hematocrit of 30% the horizontal axis in Fig. 2 denotes the measured glucose concentrations C in milligram per deciliter, and the vertical axis shows the deviation $\Delta$. For glucose concentrations below 100 mg/dL the deviations $\Delta$ are given as absolute values in mg/dL, whereas for glucose concentrations above 100 mg/dL, the deviations $\Delta$ are given as a percentage.

Such curves or polygons can be determined for a plurality of hematocrits and glucose levels, such that the curves can be put together to a hypersurface, wherein for example, the measured glucose concentration is plotted on a first axis, the hematocrit on a second axis and the deviation $\Delta$ on a third axis. Such hypersurfaces can be stored in the memory 24 for example as individual values in a lookup table or being defined analytically or in other ways, such that in each case for each hematocrit value and each measured glucose concentration, the corresponding deviation $\Delta$ can be easily deducted with the processor 22 in order to provide a corrected value of the glucose concentration. It has been found that the hematocrit dependency largely stable over different batches of test strips 12. The correction values determined are therefore generally valid for a combination of a meter 10 and a test strip 12 or other test element comprising a specific test chemistry.

**EP 2 929 336 B1**

**Claims**

1.  A method for hematocrit correction in a glucose meter (10) comprising the steps of

    a) determining by means of a reference instrument (34) preferably formed as a laboratory analyzer a hematocrit reference value of a reference blood sample taken from a specific user,
    b) applying a fresh blood sample of said user on a disposable analytical test element (12),
    c) measuring the glucose value of the fresh blood sample by single use of said test element (12) in the glucose meter (10),
    d) determining a hematocrit correction value using at least the hematocrit reference value,
    e) adjusting the measured glucose value using the hematocrit correction value to receive an adjusted glucose value,
    f) wherein glucose measurements are repeatedly conducted and corrected on the basis of the same hematocrit reference value.

2.  The method of claim 1 further comprising transferring the hematocrit reference value via a wireless or wire-bound interface (30) into a memory (24) of the glucose meter (10).

3.  The method of claim 1 or 2, wherein the hematocrit reference value is transmitted to the glucose meter (10) using a software (36) outside the glucose meter (10) which is inaccessible to the user.

4.  The method according to any of claims 1 to 3, wherein the hematocrit reference value is stored in an external database (38) outside the glucose meter (10) in connection with a user identifier for the user.

5.  The method according to any of claims 1 to 4, wherein the glucose meter (10) comprises machine readable means (40), specifically an RFID chip, for automatic user identification.

6.  The method according to any of claims 1 to 5, further comprising checking the user identity by a query provided by the glucose meter (10) and requesting input of a confirmation by the user.

7.  The method according to any of claims 1 to 6, further comprising asking the user about a change in living conditions influencing hematocrit.

8.  The method according to any of claims 1 to 7, further comprising verifying the timeliness of the hematocrit reference value within a given time interval.

9.  The method according to any of claims 1 to 8, further comprising displaying the adjusted glucose value to the user upon fulfillment of given conditions including availability of the hematocrit reference value, and otherwise displaying the measured glucose value.

10. The method according to any of claims 1 to 9, wherein the hematocrit correction value is determined in dependence of the hematocrit reference value and the measured glucose value.

11. The method according to any of claims 1 to 10, wherein determining the hematocrit correction value involves using one or more correction functions or a lookup table determined empirically or experimentally for a given design of the test element (12) and/or the glucose meter (10).

12. The method according to any of claims 1 to 11, wherein the glucose meter is construed as a handheld device usable for self-testing on the spot.

13. A glucose meter (10) adapted for hematocrit correction, comprising

    a) means (14) configured to receive at least one disposable test element (12) on which a blood sample can be applied or is applied,
    b) a detector (16) adapted for measuring a blood glucose value using the test element (12) loaded with a fresh blood sample of a specific user,
    c) an interface (30) configured to input a hematocrit reference value of a reference blood sample of said user,
    d) a processor (20;22) adapted to determine a hematocrit correction value using the hematocrit reference value and the measured glucose value and to adjust the measured glucose value using the hematocrit correction value,
e) wherein glucose measurements are repeatedly conducted and corrected on the basis of the same hematocrit reference value.

14. The glucose meter of claim 13, wherein the glucose meter is construed as a handheld device usable for self-testing on the spot.

15. A system adapted for hematocrit correction, comprising the glucose meter according to claim 13 or 14 and a reference instrument (34) preferably formed as a laboratory analyzer to determine a hematocrit reference value of a reference blood sample taken from a specific user of the glucose meter (10).

**Patentansprüche**

1. Verfahren zur Hämatokritkorrektur in einem Glukosemessgerät (10) mit folgenden Schritten

a) Bestimmen eines Hämatokrit-Referenzwerts einer von einem bestimmten Benutzer entnommenen Referenzblutprobe mittels eines vorzugsweise als Labor-Analysators ausgebildeten Referenzgeräts (34),
b) Aufbringen einer aktuellen Blutprobe des genannten Benutzers auf einem disposiblen analytischen Testelement (12),
c) Messen des Glukosewerts der aktuellen Blutprobe durch Einmalgebrauch des genannten Testelements (12) in dem Glukosemessgerät (10),
d) Bestimmen eines Hämatokrit-Korrekturwerts unter Verwendung zumindest des Hämatokrit-Referenzwerts,
e) Korrigieren des gemessenen Glukosewerts unter Verwendung des Hämatokrit-Korrekturwerts, um einen korrigierten Glukosewert zu erhalten,
f) wobei die Glukosemessungen wiederholt durchgeführt und auf der Basis desselben Hämatokrit-Referenzwerts korrigiert werden.

2. Verfahren nach Anspruch 1, weiterhin umfassend Übertragen des Hämatokrit-Referenzwerts über eine drahtlose oder drahtgebundene Schnittstelle (30) in einen Speicher (24) des Glukosemessgeräts (10).

3. Verfahren nach Anspruch 1 oder 2, bei welchem der Hämatokrit-Referenzwert unter Verwendung einer außerhalb des Glukosemessgeräts (10) befindlichen, für den Benutzer nicht zugänglichen Software (36) in das Glukosemessgerät (10) übertragen wird.

4. Verfahren nach einem der Ansprüche 1 bis 3, bei welchem der Hämatokrit-Referenzwert in einer externen Datenbank (38) außerhalb des Glukosemessgeräts (10) in Verbindung mit einem Nutzer-Identifizierer für den Benutzer gespeichert wird.

5. Verfahren nach einem der Ansprüche 1 bis 4, bei welchem das Glukosemessgerät (10) ein maschinenlesbares Mittel (40), insbesondere einen RFID Chip zur automatischen Benutzeridentifikation umfasst.

6. Verfahren nach einem der Ansprüche 1 bis 5, weiterhin umfassend Prüfen der Identität des Benutzers durch eine durch das Glukosemessgerät (10) bereitgestellte Abfrage und Anfordern einer Bestätigungseingabe durch den Benutzer.

7. Verfahren nach einem der Ansprüche 1 bis 6, weiterhin umfassend Fragen des Benutzers nach einer Änderung in Hämatokrit beeinflussenden Lebensbedingungen.

8. Verfahren nach einem der Ansprüche 1 bis 7, weiterhin umfassend Verifizieren der innerhalb eines gegebenen Zeitintervalls liegenden Aktualität des Hämatokrit-Referenzwerts.

9. Verfahren nach einem der Ansprüche 1 bis 8, weiterhin umfassend Anzeigen des korrigierten Glukosewerts für den Benutzer bei Erfüllung von die Verfügbarkeit des Hämatokrit-Referenzwerts einschließenden vorgegebenen Bedingungen und andernfalls Anzeigen des gemessenen Glukosewerts.

10. Verfahren nach einem der Ansprüche 1 bis 9, bei welchem der Hämatokrit-Korrekturwert in Abhängigkeit von dem Hämatokrit-Referenzwert und dem gemessenen Glukosewert bestimmt wird.

**11.** Verfahren nach einem der Ansprüche 1 bis 10, bei welchem die Bestimmung des Hämatokrit-Korrekturwerts umfasst Verwendung einer oder mehrerer Korrekturfunktionen oder einer Nachschlagetabelle, welche empirisch oder experimentell für ein gegebenes Design des Testelements (12) und/oder des Glukosemessgeräts (10) bestimmt wird.

**12.** Verfahren nach einem der Ansprüche 1 bis 11, bei welchem das Glukosemessgerät als ein für Selbsttests vor Ort einsetzbares Handgerät ausgebildet ist.

**13.** Zur Hämatokrit-Korrektur angepasstes Glukosemessgerät (10) mit

a) Mitteln (14) die dazu ausgelegt sind, mindestens ein disposibles Testelement (12) aufzunehmen, auf welchem eine Blutprobe aufgebracht werden kann oder aufgebracht ist,
b) einem Detektor (16), der dazu geeignet ist, einen Blutglukosewert unter Verwendung des mit einer aktuellen Blutprobe eines bestimmten Benutzers beladenen Testelements (12) zu erfassen,
c) einer Schnittstelle (30), die dazu ausgelegt ist, einen Hämatokrit-Referenzwerts einer Referenzblutprobe des genannten Benutzers einzugeben,
d) einem Prozessor (20;22), der dazu ausgelegt ist, einen Hämatokrit-Korrekturwert unter Verwendung des Hämatokrit-Referenzwerts und des gemessenen Glukosewerts zu bestimmen und den Glukosewert unter Verwendung des Hämatokrit-Korrekturwerts zu korrigieren,
e) wobei die Glukosemessungen wiederholt durchgeführt und auf der Basis desselben Hämatokrit-Referenzwerts korrigiert werden.

**14.** Glukosemessgerät nach Anspruch 13, bei welchem das Glukosemessgerät als für Selbsttests vor Ort einsetzbares Handgerät ausgebildet ist.

**15.** Zur Hämatokrit-Korrektur ausgelegtes System mit dem Glukosemessgerät nach Anspruch 13 oder 14 und mit einem vorzugsweise als Labor-Analysator ausgebildeten Referenzgerät (34), um einen Hämatokrit-Referenzwert einer von einem bestimmten Benutzer des Glukosemessgeräts (10) entnommenen Referenzblutprobe zu bestimmen.

**Revendications**

**1.** Procédé pour la correction de l'hématocrite dans un lecteur de glycémie (10), comprenant les étapes dans lesquelles :

a) on détermine, au moyen d'un instrument de référence (34), de préférence réalisé sous la forme d'un analyseur de laboratoire, une valeur de référence de l'hématocrite d'un échantillon de sang de référence prélevé auprès d'un utilisateur spécifique ;
b) on applique un échantillon de sang frais dudit utilisateur sur un élément de test diagnostique jetable (12) ;
c) on mesure la valeur de glycémie de l'échantillon de sang frais via une utilisation unique dudit élément de test (12) dans le lecteur de glycémie (10) ;
d) on détermine une valeur de correction de l'hématocrite en utilisant au moins la valeur de référence de l'hématocrite ;
e) on ajuste la valeur de glycémie mesurée en utilisant la valeur de correction de l'hématocrite pour obtenir une valeur de glycémie ajustée ;
f) dans lequel on effectue les mesures de la glycémie de manière répétée et on les corrige sur base de la même valeur de référence de l'hématocrite.

**2.** Procédé selon la revendication 1, comprenant en outre le fait de transférer la valeur de référence de l'hématocrite via une interface sans fil ou câblée (30) dans une mémoire (24) du lecteur de glycémie (10).

**3.** Procédé selon la revendication 1 ou 2, dans lequel la valeur de référence de l'hématocrite est transmise au lecteur de glycémie (10) en utilisant un logiciel (36) en dehors du lecteur de glycémie (10), qui est inaccessible pour l'utilisateur.

**4.** Procédé selon l'une quelconque des revendications 1 à 3, dans lequel la valeur de référence de l'hématocrite est stockée dans une base de données externe (38) en dehors du lecteur de glycémie (10) en liaison avec un identifiant pour l'utilisateur.

**5.** Procédé selon l'une quelconque des revendications 1 à 4, dans lequel le lecteur de glycémie (10) comprend un moyen lisible par machine (40), spécifiquement une puce RFID, pour une identification automatique de l'utilisateur.

6.  Procédé selon l'une quelconque des revendications 1 à 5, comprenant en outre le fait de vérifier l'identité de l'utilisateur sur base d'une demande d'information émise par le lecteur de glycémie (10) et le fait de réclamer une entrée de confirmation par l'utilisateur.

7.  Procédé selon l'une quelconque des revendications 1 à 6, comprenant en outre le fait de questionner l'utilisateur quant à des changements de ses conditions de vie qui influencent l'hématocrite.

8.  Procédé selon l'une quelconque des revendications 1 à 7, comprenant en outre le fait de vérifier l'actualité de la valeur de référence de l'hématocrite dans un intervalle de temps donné.

9.  Procédé selon l'une quelconque des revendications 1 à 8, comprenant en outre le fait d'afficher la valeur de glycémie ajustée à l'utilisateur lorsque des conditions données sont réunies, notamment la disponibilité de la valeur de référence de l'hématocrite, et sinon le fait d'afficher la valeur de glycémie mesurée.

10. Procédé selon l'une quelconque des revendications 1 à 9, dans lequel on détermine la valeur de correction de l'hématocrite en fonction de la valeur de référence de l'hématocrite et de la valeur de glycémie mesurée.

11. Procédé selon l'une quelconque des revendications 1 à 10, dans lequel la détermination de la valeur de correction de l'hématocrite implique l'utilisation d'une ou plusieurs fonctions de correction ou d'une table de référence déterminée par voie empirique ou par voie expérimentale pour un type donné de l'élément de test (12) et/ou du lecteur de glycémie (10).

12. Procédé selon l'une quelconque des revendications 1 à 11, dans lequel le lecteur de glycémie est réalisé sous la forme d'un dispositif tenu en main que l'on peut utiliser pour se tester soi-même sur place.

13. Lecteur de glycémie (10) conçu pour une correction de l'hématocrite, comprenant :

    a) un moyen (14) configuré pour recevoir au moins un élément de test jetable (12) sur lequel on peut appliquer ou on applique un échantillon de sang ;
    b) un détecteur (16) conçu pour mesurer une valeur de glycémie en utilisant l'élément de test (12) chargé avec un échantillon de sang frais d'un utilisateur spécifique ;
    c) une interface (30) configurée pour entrer une valeur de référence de l'hématocrite d'un échantillon de sang de référence dudit utilisateur ;
    d) un processeur (20, 22) conçu pour déterminer une valeur de correction de l'hématocrite en utilisant la valeur de référence de l'hématocrite et la valeur de glycémie mesurée afin d'ajuster la valeur de glycémie mesurée en utilisant la valeur de correction de l'hématocrite ;
    e) dans lequel on effectue les mesures de la glycémie de manière répétée et on les corrige sur base de la même valeur de référence de l'hématocrite.

14. Lecteur de glycémie selon la revendication 13, dans lequel le lecteur de glycémie est réalisé sous la forme d'un dispositif tenu en main que l'on peut utiliser pour se tester soi-même sur place.

15. Système conçu pour une correction de l'hématocrite, comprenant le lecteur de glycémie selon la revendication 13 ou 14 et un instrument de référence (34) de préférence réalisé sous la forme d'un analyseur de laboratoire pour déterminer une valeur de référence de l'hématocrite d'un échantillon de sang de référence prélevé auprès d'un utilisateur spécifique du lecteur de glycémie (10).

Fig. 1

C [mg/dL]

Fig. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2012103806 A1, Shinno Teppei

- US 2004225205 A1, Ilya Fine